# EUROPÄISCHE PATENTANMELDUNG

(11) **EP 4 223 247 A1**
(43) Veröffentlichungstag der Anmeldung: **09.08.2023**
(21) Anmeldenummer: 23155164.9
(22) Anmeldetag: 06.02.2023
(51) Int. Cl.: A61B 34/30, A61B 90/00

(54) **STERILEINHEIT UND MANIPULATOR FÜR DIE ROBOTISCHE CHIRURGIE**

(30) Priorität: 07.02.2022 DE 102022102806
(71) Anmelder: avateramedical GmbH, 07745 Jena (DE)
(72) Erfinder: MITZLAFF, Lothar, 8600-531 Lagos (PT); MEYER, Anne-Ruth, 98693 Ilmenau (DE)
(74) Vertreter: Gleim Petri Patent- und Rechtsanwaltspartnerschaft mbB

(57) **Zusammenfassung**

Die Erfindung betrifft eine Sterileinheit (10) zum sterilen Verbinden einer Instrumentenantriebseinheit (20) mit einem chirurgischen Instrument (30), umfassend eine Basis (11) mit mindestens einer kreisförmigen Durchgangsöffnung, wobei in der Durchgangsöffnung ein Übertragungselement (12) angeordnet ist, welches um eine Drehachse (A) drehbar gelagert und in Richtung der Drehachse axial in einem vorgegebenen Bewegungsbereich zwischen einer ersten und einer zweiten Endlage beweglich ist, und einen ersten Befestigungsmechanismus, mit dem die Sterileinheit (10) mit der Instrumentenantriebseinheit (20) verbunden wird, und einen zweiten Befestigungsmechanismus (14), mit dem die Sterileinheit (10) mit dem Instrument (30) verbunden wird. Um die Kopplung zwischen der Sterileinheit (10) und der Instrumentenantriebseinheit (20) zu verbessern, weist das Übertragungselement (12) an einer Umlauffläche und die Durchgangsöffnung an einer Durchgangswand jeweils einen zueinander koaxial ausgerichteten und zueinander komplementären konischen Bereich auf, die in der ersten Endlage form- und kraftschlüssig in Kontakt stehen, so dass eine Drehung des Übertragungselements (12) um die Drehachse relativ zu der Basis (11) gehemmt wird.

## Beschreibung

### Gebiet der Erfindung

Die Erfindung betrifft eine Sterileinheit zum sterilen Verbinden einer Instrumentenantriebseinheit mit einem chirurgischen Instrument, welche eine Basis mit mindestens einer kreisförmigen Durchgangsöffnung umfasst, wobei in der Durchgangsöffnung ein Übertragungselement angeordnet ist, welches um eine Drehachse drehbar gelagert und in Richtung der Drehachse axial in einem vorgegebenen Bewegungsbereich zwischen einer ersten und einer zweiten Endlage beweglich ist. Ferner umfasst die Sterileinheit einen ersten Befestigungsmechanismus, mit dem die Sterileinheit mit der Instrumentenantriebseinheit verbunden wird, und einen zweiten Befestigungsmechanismus, mit dem die Sterileinheit mit dem Instrument verbunden wird. Mittels der Übertragungselemente wird ein Drehmoment von der Instrumentenantriebseinheit auf das chirurgische Instrument übertragen. Die Sterileinheit ist dabei als Teil einer Sterilbarriere ausgebildet und schirmt so den Operationsbereich, in dem das chirurgische Instrument arbeitet, von der nicht-sterilen Instrumentenantriebseinheit ab.

Ferner betrifft die Erfindung einen Manipulator für die robotische Chirurgie, der eine Instrumentenantriebseinheit und eine Sterileinheit zum Ankoppeln eines chirurgischen Instruments umfasst, wobei die Sterileinheit eine Basis mit mindestens einem Übertragungselement aufweist, das in einer dem mindestens einen Übertragungselement zugeordneten kreisförmigen Durchgangsöffnung angeordnet, um eine Drehachse drehbar gelagert und in Richtung der Drehachse axial in einem definierten Bewegungsbereich zwischen einer ersten und einer zweiten Endlage beweglich ist. Ferner umfasst der Manipulator einen ersten Befestigungsmechanismus, mit dem die Sterileinheit mit der Instrumentenantriebseinheit verbunden ist, wobei die Instrumentenantriebseinheit einen oder mehrere Antriebe aufweist und dem mindestens einen Übertragungselement ein Antrieb zugeordnet ist. Der Antrieb weist zudem ein Eingriffselement und das mindestens eine Übertragungselement eine zu dem Eingriffselement komplementäre Eingriffsstruktur auf. Bei dem Eingriffselement kann es sich zum Beispiel um einen oder mehrere Zapfen handeln, die Eingriffsstruktur umfasst dann eine zu dem einen oder mehreren Zapfen passsende Nuten. Auch andere geeignete, fachübliche Strukturen sind hier denkbar. Das Übertragungselement befindet sich in der zweiten Endlage, wenn das Übertragungselement und das Eingriffselement rotatorisch um die Drehachse so zueinander ausgerichtet sind, dass das Eingriffselement in die Eingriffsstruktur greift und ein an dem Eingriffselement senkrecht zur Drehachse wirkendes Drehmoment auf die Eingriffsstruktur überträgt. Das Übertragungselement befindet sich in der ersten Endlage, wenn das Eingriffselement und die Eingriffsstruktur nicht zueinander ausgerichtet sind. In diesem Fall liegt das Übertragungselements an der Unterseite auf dem Eingriffselement auf und wird so axial zu der Drehachse angehoben.

### Stand der Technik

Telemanipulatoren werden seit vielen Jahrzehnten erfolgreich in den verschiedensten Umgebungen eingesetzt, die ein Mensch nicht ohne weiteres erreichen kann. Dies betrifft Weltraumanwendungen, Unterwasseranwendungen, Atomreaktoren und vor allem auch chirurgische Anwendungen.

In den vergangenen zwei Jahrzehnten hat sich die Disziplin der laparoskopischrobotischen Chirurgie etabliert, welche Konzepte der Robotik mit denen der virtuellen Realität verbindet, so dass ein Arzt minimalinvasive Eingriffe am Patienten unter Ausnutzung seiner Augen-Hand-Koordination durchführen kann.

Bei der manuell-laparoskopischen Chirurgie werden Instrumente eingesetzt, welche einen langen Schaft besitzen, an dessen distalen Ende sich ein Endeffektor, z.B. Greifer oder Schere, und an dessen proximalen Ende sich ein Handgriff befindet. Letzterer weist einen Hebel, Knopf oder einen ähnlichen Mechanismus auf, mit dessen Hilfe der Endeffektor bewegt werden kann. Die Handhabung solcher Instrumente gestaltet sich schwierig, da sie durch einen kleinen Einschnitt am Patienten geführt in ihrer Bewegungsfreiheit stark eingeschränkt sind. Auch ist es schwierig für den Arzt, sich zu orientieren, da ein in der Regel abweichender Blickwinkel des Endoskops kompensiert werden muss.

Diese Schwierigkeit kann durch einen Telemanipulator gelöst werden. Ein solcher besteht aus mehreren Roboterarmen, den Manipulatoren, und in der Regel zwei Eingabegeräten, eines für jede Hand. Einer der Manipulatoren hält und steuert ein Endoskop, die anderen jeweils ein chirurgisches Instrument. Durch entsprechende Transformationen kann ein mit den Manipulatoren und Eingabegeräten verbundener Computer für den Chirurgen den Eindruck schaffen, dass er aus Richtung des Endoskops auf die Operationsstelle schaut und die Endeffektoren der Instrumente seine Hände sind. Auf diese Weise kann er seine Augen-Hand-Koordination ausnutzen und effektiv arbeiten.

Während im OP-Saal die Eingabegeräte eines Telemanipulators in der Regel von dem Patienten entfernt angeordnet sind, müssen die Manipulatoren nah an den Patienten heran. Dieser Bereich ist jedoch zwingend steril zu halten, um eine Kontamination der Wunden des Patienten mit Keimen möglichst zu verhindern. Dazu werden die Manipulatoren mittels aus Kunststoff gefertigter Sterilfolien verpackt. Dies ist für die Instrumente jedoch nicht möglich, so dass diese meist direkt sterilisiert werden. Dabei kommen zum einen Instrumente zum Einsatz, die nach ihrer Verwendung erneut sterilisiert werden können, oder sie werden nach einmaliger Nutzung entsorgt.

Im Stand der Technik hat sich daher etabliert, Manipulatoren von chirurgischen Robotersystemen mit einer Instrumentenantriebseinheit auszustatten, welche mit Sterilfolie verpackt wird. Um nun die Instrumente mit dieser betrieben zu können, werden Steriladapter eingesetzt, die mittels Übertragungselementen, die als Drehscheiben ausgebildet sind, eine Kraft von den Antrieben der Instrumentenantriebseinheit auf die Abtriebe des Instruments übertragen. Diese Komponenten werden in der Regel formschlüssig miteinander gekoppelt, was wiederum ein Ausrichten der Übertragungselemente zu den Antrieben und schließlich zu den Abtrieben des Instruments erfordert.

Eine Lösung für dieses Problem ist aus der EP 3 119 328 B1 bekannt, welche einen Steriladapter offenbart, der mehrere, drehbar gelagerte Übertragungselemente aufweist. Um mit den Antrieben der Instrumentenantriebseinheit in Verbindung zu kommen, weisen die Übertragungselemente zum Eingriff in die Antriebe ausgebildete Vorsprünge auf. Ferner umfassen die Übertragungselemente jeweils eine Aussparung, die mit einem Verriegelungsmechanismus in Eingriff kommt, der die Übertragungselemente in ihrer Ausrichtung fixiert. Ein Koppeln des Steriladapters mit der Instrumentenantriebseinheit bewirkt dann, dass die Übertragungselemente angehoben werden, so dass sie entweder an dem Verriegelungsmechanismus von unten anliegen oder die Verriegelungselemente sofort in die Aussparungen greifen. In beiden Fällen drehen anschließend die Antriebe, wobei im ersten Fall die Übertragungselemente durch Reibung mitgenommen werden, bis sie in den Verriegelungsmechanismus greifen können und blockieren. Die Antriebe drehen so lange, bis das Übertragungselement zum Antrieb ausgerichtet ist, also bis die Vorsprünge und die jeweiligen Aussparungen übereinanderliegen, und das Übertragungselement mit dem jeweiligen Antrieb in Formschluss fällt, so dass ein Drehmoment übertragen wird.

Die JP 2020-162637 A2 offenbart eine Sterileinheit für ein chirurgisches System, welche zwischen einer Instrumentenantriebseinheit und einem chirurgischen Instrument angeordnet werden kann. Sowohl auf Seiten des Instruments als auch auf Seiten der Instrumentenantriebseinheit befinden sich Übertragungsstrukturen, durch die eine Kraft zum Aktuieren, also zum robotergestützten Auslenken eines Endeffektors des Instruments, mittels Übertragungselementen der Sterileinheit übertragen wird. In dieser Lösung muss jeder Antrieb der Instrumentenantriebseinheit und ein ihm zugehöriges Übertragungselement zueinander ausgerichtet werden. Dazu wird ein Unterschied zwischen einem Reibmoment, welches an einer ersten Kontaktfläche wirkt, und einem an einer zweiten Kontaktfläche wirkenden Reibmoment ausgenutzt. Die erste Kontaktfläche ist an der der Instrumentenantriebseinheit abgewandten Seite des Übertragungselements und berührt eine Basis der Sterileinheit an einer umlaufenden Kante. Die zweite Kontaktfläche ist an der der Instrumentenantriebseinheit zugewandten Seite angeordnet und berührt, sofern das Übertragungselement und der zugehörige Antrieb nicht zueinander ausgerichtet sind, eine Eingriffsstruktur an dem Antrieb. Ein an der ersten Kontaktfläche wirkendes Reibmoment ist dabei größer als das an der zweiten Kontaktfläche wirkende. Auf diese Weise wird eine Drehung des Übertragungselements gehemmt und der Antrieb kann zum Übertragungselement ausgerichtet werden. Nach dem Ausrichten greift das Eingriffselement in die Eingriffsstruktur und das Übertragungselement senkt sich ab, so dass die erste Kontaktfläche die Basis der Sterileinheit nicht mehr berührt. Ein Nachteil dieser Lösung ist, dass es bei einem solchen Aufbau aufgrund der umlaufenden Kante an der Sterileinheit dazu kommen kann, dass sich das Übertragungselement in der Basis verklemmt und im weiteren Betrieb beschädigt wird, sollte die Sterileinheit nicht gerade auf die Instrumentenantriebseinheit aufgesetzt werden. Ferner kann es vorkommen, dass je nach Materialkombination und Anpressdruck, der an den Kontaktflächen wirkt, das Übertragungselement trotzdem langsam um die eigene Achse rotiert, so dass der oben beschriebene Ausrichtungsprozess länger dauert.

### Beschreibung der Erfindung

Es ist daher Aufgabe der Erfindung, eine Sterileinheit und einen Manipulator für ein chirurgisch robotisches System bereitzustellen, die es ermöglichen, die Übertragungselemente leichter verbinden zu können, und das Ausrichten der Übertragungselemente und Antriebe zu erleichtern.

Die Aufgabe wird bei der eingangs beschriebenen Sterileinheit dadurch gelöst, dass das Übertragungselement an einer Umlauffläche und die Durchgangsöffnung an einer Durchgangswand jeweils einen zueinander koaxial ausgerichteten und zueinander komplementären konischen Bereich aufweisen, die in der ersten Endlage form- und kraftschlüssig in Kontakt stehen, so dass eine Drehung des Übertragungselements um die Drehachse relativ zu der Basis gehemmt wird. Die konischen Bereiche wirken dabei selbsthemmend, so dass ein erhöhtes Reibmoment erzeugt wird. Das Reibmoment ist abhängig von einer die beiden Körper zusammendrückenden, senkrecht auf deren Oberfläche wirkenden Normalkraft und einem Reibungskoeffizienten zwischen den Körpern als Materialkonstante. Bei der konischen Form kommt es beim Einfügen des Übertragungselements zu einer umlaufenden elastischen Verformung sowohl des Übertragungselements als auch der Basis an dem konischen Bereich, da die zusammenführende Kraft unter einem Winkel zur Oberfläche wirkt. Die dieser Verformung entgegenwirkende Kraft ist für die Selbsthemmung verantwortlich, denn sie erhöht die wirkende Normalkraft und so das Reibmoment. Die konischen Bereiche wirken ferner als eine Führung des Übertragungselements, welche das Verklemmen des Übertragungselements verhindert. Gleichzeitig können die Übertragungselemente einstückig gefertigt werden, so dass der Steriladapter aus weniger Bauteilen zusammengesetzt sein kann und somit Herstellungskosten reduziert werden können.

In einer vorteilhaften Ausgestaltung verjüngen sich die konischen Bereiche derart, dass die konischen Bereiche jeweils einen Kegelstumpf eines Kegels bilden, wobei der Kegel einen Öffnungswinkel zwischen 10° und 60° aufweist. Auf diese Weise wird eine wirkende Normalkraft vergrößert und so die Selbsthemmung optimiert.

Dabei ist es ferner vorteilhaft, den konischen Bereich des Übertragungselements und/oder den konischen Bereich der Durchgangsöffnung aufzurauen oder mit einem den Reibungskoeffizienten vergrößernden Material zu versehen, um das Übertragungselement besser zu hemmen. Bei der Reibung fester Körper wirken primär zwei Effekte: die Rauheit, die mikroskopische Formschlüsse bewirkt, wenn die festen Körper übereinander gleiten, sowie die Adhäsion, also Kraftschluss durch molekulare Anziehungskräfte. Beides kann durch entsprechende Wahl der Materialien, eine Bearbeitung der Oberflächen oder eine Beschichtung dieser mit geeigneten Materialien beeinflusst werden. Eine Besonderheit besteht bei der Gummierung der Oberfläche oder dem Einbringen eines Gummiringes, da die Reibung bei Gummi sehr stark von der inneren Reibung eines Elastomers abhängt, aus dem der Gummi besteht. Bei einem Kontakt zwischen einem Elastomer und einer starren Oberfläche wird die Reibungsenergie nämlich durch eine Deformation des Elastomers dissipiert.

In einer weiteren vorteilhaften Ausgestaltung wird das Übertragungselement durch mindestens zwei Schnapphaken in der Durchgangsöffnung des Sterileinheit gehalten. Dies erleichtert die Montage weiter und reduziert die Herstellungskosten. Ferner wird so der zur Drehachse axiale Bewegungsbereich auf besonders einfache Weise realisiert, indem ein Abstand zwischen den Schnapphaken und einem den Schnapphaken gegenüberliegenden Vorsprung erreicht wird.

Die Aufgabe wird au ßerdem bei dem eingangs beschriebenen Manipulator dadurch gelöst, dass das Übertragungselement an einer Umlauffläche und die Durchgangsöffnung an einer Durchgangswand jeweils einen zueinander koaxial ausgerichteten und zueinander komplementären konischen Bereich aufweisen, wobei die konischen Bereiche in der ersten Endlage formschlüssig und kraftschlüssig in Kontakt kommen, wodurch eine Drehung des Übertragungselements um die Drehachse relativ zu der Basis gehemmt wird. Bei Verbinden der Sterileinheit mit der Instrumentenantriebseinheit muss, bevor das Instrument angesteuert werden kann, das Eingriffselement in die Eingriffsstruktur greifen. Sobald die Sterileinheit mittels Befestigungselement an der Instrumentenantriebseinheit befestigt ist, drehen die Antriebe das Eingriffselement um die Drehachse. Da sich das Übertragungselement hier in der zweiten Endlage befindet und eine Drehung relativ zur Basis durch Form- und Kraftschluss der konischen Bereiche gehemmt wird, gleitet das Eingriffselement über die Unterseite des Übertragungselements. Sobald das Eingriffselement und die Eingriffsstruktur zueinander ausgerichtet sind, fällt das Übertragungselement auf das Eingriffselement, so dass es zum Eingriff kommt und im Folgenden ein am Antrieb erzeugtes Drehmoment auf das Übertragungselement wirkt und im Anschluss durch letzteres auf das Instrument übertragen wird.

In einer vorteilhaften Ausgestaltung verjüngen sich die konischen Bereiche derart, dass die konischen Bereiche jeweils einen Kegelstumpf eines Kegels bilden, wobei der Kegel einen Öffnungswinkel zwischen 10° und 60° aufweist. Auf diese Weise wird die wirkende Normalkraft vergrößert und so die Selbsthemmung optimiert.

In einer weiteren vorteilhaften Ausgestaltung ist der Reibungskoeffizient zwischen dem konischen Bereich des Übertragungselements und dem konischen Bereich der Durchgangsöffnung größer als der Reibungskoeffizient zwischen einer Unterseite des Übertragungselements und dem Eingriffselement. In der zweiten Endlage wird der konische Bereich des Übertragungselements auf den konischen Bereich der Durchgangsöffnung gepresst. Um auf diese Weise die Drehung des Übertragungselements um die eigene Achse zu hemmen, müssen sich die Reibungskoeffizienten hinreichend unterscheiden, dass sich das Übertragungselement entweder gar nicht bewegt, während der Antrieb das Eingriffselement ausrichtet, oder das Übertragungselement sich zumindest langsamer als der Antrieb dreht.

Um den Reibungskoeffizienten anzupassen, ist es besonders vorteilhaft, wenn der konische Bereich des Übertragungselements und/oder der konische Bereich der Durchgangsöffnung aufgeraut oder mit einem den Reibungskoeffizienten vergrößernden Material versehen sind. Wie bereits oben näher dargelegt, wird auch in dieser vorteilhaften Ausführung das Übertragungselement stärker gehemmt.

Auch für diese erfindungsgemäße Lösung ist es vorteilhaft, wenn das Übertragungselement durch mindestens zwei Schnapphaken in der Durchgangsöffnung gehalten wird, da so die Montage erleichtert und Herstellungskosten reduziert werden können.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

### Kurze Beschreibung der Zeichnungen

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Es zeigen:
- Fig. 1: einen Manipulator mit einer Instrumentenantriebseinheit, einer Sterileinheit in erster Ausführung und ein chirurgisches Instrument,
- Fig. 2: die Instrumentenantriebseinheit, die Sterileinheit in erster Ausführung und das chirurgische Instrument in einer Explosionsdarstellung,
- Fig. 3A: eine zweite Ausführung der Sterileinheit in einer ersten Ansicht,
- Fig. 3B: eine zweite Ausführung der Sterileinheit in einer zweiten Ansicht,
- Fig. 3C: ein Übertragungselement in einer Detaildarstellung,
- Fig. 4: eine Schnittdarstellung der zweiten Ausführung der Sterileinheit.

### Ausführliche Beschreibung der Zeichnungen

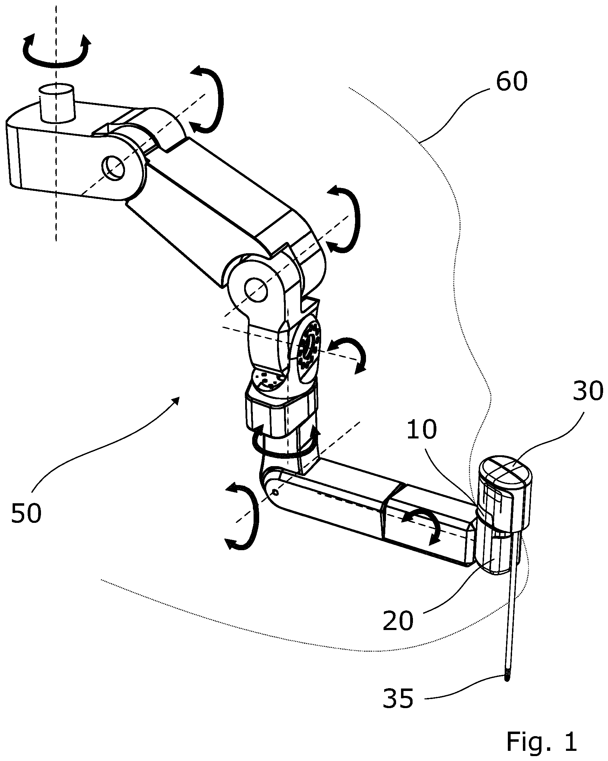

Fig.1 zeigt einen robotischen Manipulator 50 mit einer Instrumentenantriebseinheit 20 und ein Instrument 30. Derartige Manipulatoren 50 umfassen eine Vielzahl von Gelenken, die eine freie Positionierung des Instruments 30 im Raum ermöglichen. Dabei können sie eigenständig als Hilfe für den Chirurgen, z.B. als Endoskophalterungen, oder im Rahmen eines chirurgischen Telemanipulators eingesetzt werden. Da der Bereich um einen zu operierenden Patienten immer steril sein muss, um Infektionen zu vermeiden, werden schwer zu sterilisierende Teile des Systems, also der Manipulator 50 und die Instrumentenantriebseinheit 20, durch eine Sterilbarriere 60 vom sterilen Bereich abgetrennt. Um trotzdem einen Endeffektor 35 des Instruments 30 aktuieren zu können, ist die Sterileinheit 10, hier in einer ersten Ausführung gezeigt, wie oben beschrieben als Adapter ausgebildet.

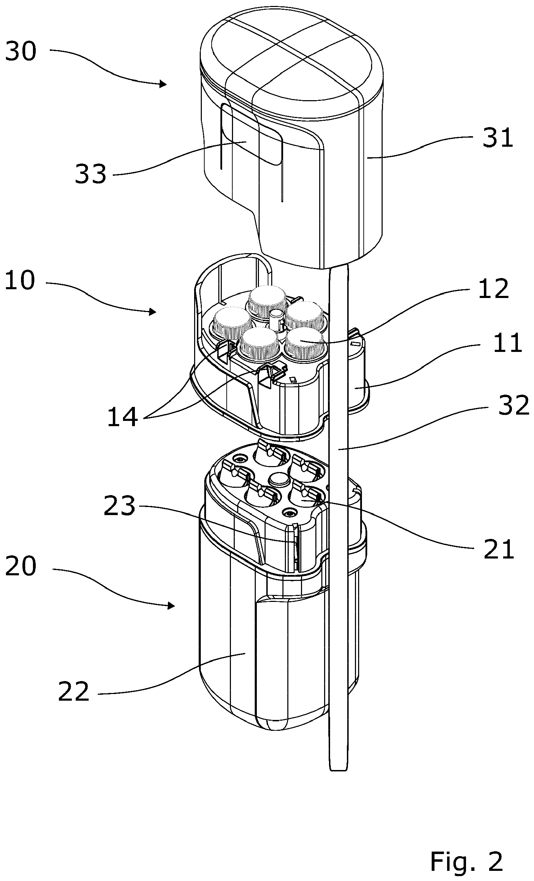

Fig. 2 stellt dar, wie das Instrument 30, die erste Ausführung der Sterileinheit 10 und die Instrumentenantriebseinheit 20 zueinander angeordnet werden. Die Sterileinheit 10 weist eine Basis 11 und mehrere Übertragungselemente 12 auf, die drehbar um eine Drehachse gelagert sind. Die Sterileinheit 10 wird zwischen Instrument 30 und Instrumentenantriebseinheit 20 angeordnet.

Das chirurgische Instrument 30 setzt sich aus einem Instrumentengehäuse 31 und einem Instrumentenschaft 32 zusammen. An einem distalen Ende des Instrumentenschafts 32 ist hier kein Endeffektor 35 angeordnet, da es sich um ein Endoskop handelt. Allgemein können aber sämtliche Typen Instrumenten 30 mit den entsprechenden Endeffektoren 35 eingesetzt werden, so zum Beispiel Greifer, Scheren, Klammernahtinstrumente, Dissektoren. Die Anzahl der Freiheitsgrade ist dabei abhängig vom Typ des Instruments 30, wobei neben der Bewegung von Endeffektorgelenken auch eine mechanische Auslösung von zusätzlichen Funktionen, beispielsweise dem Aktivieren einer Klinge, umfasst sind. In der Regel liegt die Anzahl der Freiheitsgrade zwischen einem einzelnen für zum Beispiel ein Endoskop oder fünf für ein Klammernahtinstrument, prinzipiell ist sie jedoch beliebig und kann an den konkreten Anwendungsfall angepasst werden.

Die Instrumentenantriebseinheit 20 umfasst ein Antriebsgehäuse 22, in dem fünf Antriebe 21 untergebracht sind, welche jeweils ein Eingriffselement und einen hier nicht sichtbaren Motor aufweisen, wobei es sich in der Regel um Elektromotoren handelt, welche ein Drehmoment erzeugen. Das Eingriffselement ist dabei außerhalb des Antriebsgehäuses 22 angeordnet, eine genauere Beschreibung erfolgt weiter unten. Das Drehmoment wird mittels des jeweiligen Eingriffselements auf die Übertragungselemente 12 übertragen, die hierzu eine jeweils komplementäre Eingriffsstruktur aufweisen. Auf der dem Instrument 30 zugewandten Seite sind die Übertragungselemente 12 mit einer beliebigen Koppelstruktur versehen, mit der das Drehmoment an hier verdeckte Abtriebe des Instruments 30 weitergegeben wird, um den Endeffektor 35 zu bewegen. Die Koppelstruktur ist im vorliegenden Beispiel als geriffelte Umlauffläche des jeweiligen Übertragungselements 12 ausgebildet. Im Stand der Technik werden jedoch auch andere Varianten eingesetzt, die auch allesamt hier an Stelle der Koppelstruktur Anwendung finden können.

Die Instrumentenantriebseinheit 20 umfasst neben den Antrieben 21 und dem Antriebsgehäuse 22 ein oder mehrere erste Befestigungselemente 23, welche die Sterileinheit 10 an der Instrumentenantriebseinheit 20 befestigen. Dazu ist an der Sterileinheit 10 wiederum ein komplementärer, in dieser Abbildung verdeckter, erster Befestigungsmechanismus vorhanden. Auf der dem Instrument 30 zugewandten Seite der Sterileinheit 10 befindet sich ein zweiter Befestigungsmechanismus 14, welcher wiederum ermöglicht, das chirurgische Instrument 30 an der Sterileinheit 10 zu befestigen. Diese Verbindung kann mittels eines Auslösemechanismus 33 gelöst werden.

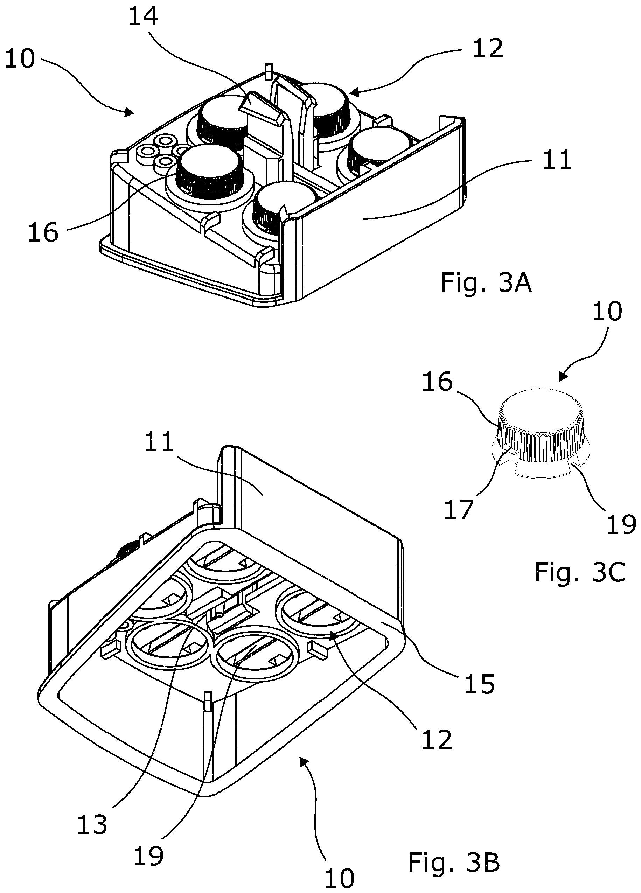

In den Fig. 3A und 3B ist die Sterileinheit 10 genauer in einer zweiten Ausführung dargestellt. Außer den Übertragungselementen 12 und der Basis 11 sind an der Sterileinheit 10 ein erster Befestigungsmechanismus 13 und ein zweiter Befestigungsmechanismus 14 angebracht. An der der Instrumentenantriebseinheit 20 zugeordneten Seite der Sterileinheit 10, die in Fig. 3B dargestellt ist, befindet sich der erste Befestigungsmechanismus 13, welcher ein Befestigen der Sterileinheit 10 an der Instrumentenantriebseinheit 20 mittels der dort befindlichen, ersten Befestigungselemente 23 ermöglicht. Auf der dem Instrument 30 zugewandten Seite der Sterileinheit 10, die in Fig. 3A eingesehen werden kann, befindet sich der zweite Befestigungsmechanismus 14, welcher ermöglicht, das chirurgische Instrument 30 an der Sterileinheit 10 über an dem Instrument 30 befindliche, zweite Befestigungselemente zu befestigen. Beide Befestigungsmechanismen 13, 14 sind Teile jeweils einer Schnappverbindung, allgemein kann der erste und der zweite Befestigungsmechanismus 13, 14 jedoch beliebig fachmännisch gestaltet werden, sofern die Sterileinheit 10 mit dem Instrument 30 und der Instrumentenantriebseinheit 20 durch Kraft- oder Formschluss lösbar oder unlösbar verbunden werden kann.

Das Übertragungselement 12 ist genauer in Fig. 3C dargestellt und wird in eine Durchgangsöffnung der Basis 11 eingebracht, wobei es verschiedene Möglichkeiten zur konkreten Ausgestaltung gibt. So kann die Basis 11 zweiteilig gestaltet werden, so dass das Übertragungselement 12 zwischen den beiden Teilen der Basis 11 aufgenommen wird. Alternativ ist, wie in Fig. 3A, 3B und 3C gezeigt, das Übertragungselement 12 selbst mit einer Möglichkeit zur Befestigung ausgestattet. Dabei kann es selbst aus zwei Körpern zusammengesetzt sein, welche die Basis 11 zwischen sich einklemmen, oder aber mit Befestigungsmitteln ausgestattet sein, die es an der Basis 11 um die Drehachse drehbar gelagert halten. Dafür geeignet sind die in Fig. 3C zu sehenden Schnapphaken 17, Kugelgelenkverbindungen oder ähnliche Befestigungsmittel.

Nicht dargestellt aber ebenso möglich sind weitere, anders geformte Übertragungselemente 12 oder ein elektrischer Kontakt zum Leiten von Stromsignalen durch die Sterileinheit 10. Ferner ist an der der Instrumentenantriebseinheit 20 zugewandten Seite der Basis 11 der Sterileinheit 10 vorzugsweise eine Befestigungsfläche 15 vorhanden. Hier wird eine Sterilfolie mittels Kleben oder Schweißen angebracht, welche zusammen mit der Sterileinheit 10 eine Sterilbarriere 60 formt. Eine weitere fachübliche Möglichkeit, die Sterilfolie einzubringen, besteht darin, die Basis 11 zweiteilig zu gestalten, so dass die Sterilfolie zwischen die zwei Teile geklemmt wird.

Die Übertagungselemente 12 weisen an ihrer Oberseite eine Koppelstruktur 16 auf, die der Ausführungsform in Fig. 2 entspricht. An der in Fig. 3B einsehbaren Unterseite haben die Übertragungselemente 12 eine Eingriffsstruktur 19 zur Aufnahme eines Eingriffselements des zugehörigen Antriebs 21.

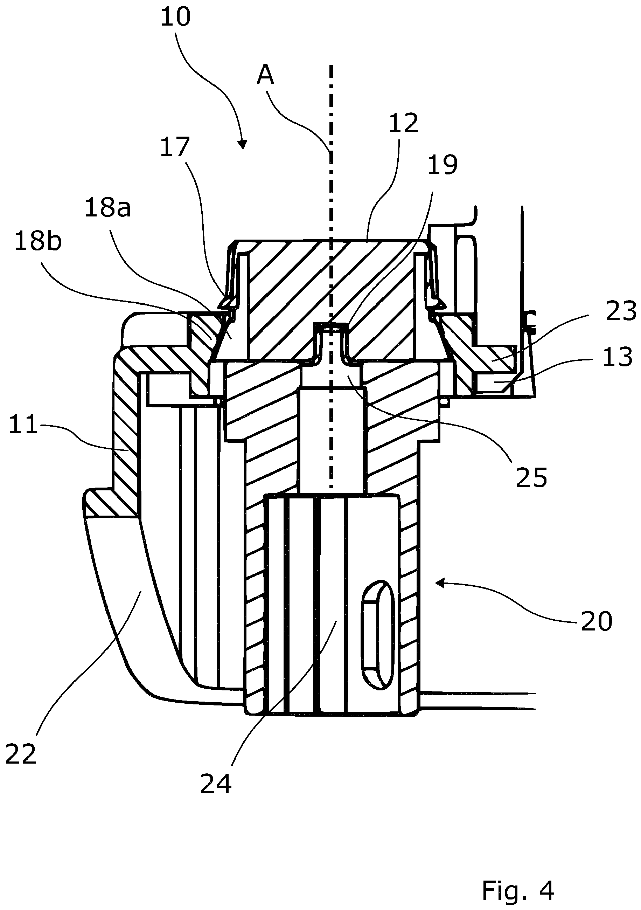

Fig. 4 zeigt einen Schnitt durch eine mit der Sterileinheit 10 verbundene Instrumentenantriebseinheit 20 an der Stelle eines Übertragungselements 12. Die Sterileinheit 10 ist mittels des ersten Befestigungsmechanismus 13 an dem ersten Befestigungselement 23 der Instrumentenantriebseinheit 20 befestigt. Es handelt sich im vorliegenden Beispiel um eine Schnappverbindung. Zwischen der Basis 11 und dem Antriebsgehäuse 22 ist eine hier nicht gezeigte Sterilfolie 60 angeordnet, die mit der Sterileinheit 10 verbunden ist. Das Übertragungselement 12 ist in einer Durchgangsöffnung angebracht und wird an der oberen Seite durch die Schnapphaken 17 gehalten. Auf der gegenüberliegenden, der Instrumentenantriebseinheit 20 zugewandten Seite weist das Übertragungselement 12 an einer Umlauffläche einen ersten konischen Bereich 18a auf. Ebenso befindet sich an der Basis 11 ein zweiter konischer Bereich 18b an einer Durchgangswand der Durchgangsöffnung. Die beiden konischen Bereiche 18a, 18b sind koaxial zueinander ausgerichtet und komplementär, so dass das Übertragungselement 12 an dieser Stelle plan mit der Basis 11 in Formschluss treten kann.

Das Übertragungselement 12 ist in Richtung einer Drehachse A axial in einem vorgegebenen Bewegungsbereich zwischen einer ersten und einer zweiten Endlage beweglich. Die Endlagen sind abhängig von der Ausrichtung der Eingriffsstruktur 19 zu einem Eingriffselement 25. Das Eingriffselement 25 ist hier als quaderförmiger Zapfen ausgebildet und die Eingriffsstruktur 19 als eine zu dem Zapfen passsende Nut. Auch andere geeignete, fachübliche Strukturen sind hier denkbar. So sind mehrere Zapfen und dazu komplementäre Nuten genauso denkbar wie Strukturen, die kreuzförmig, sternförmig oder vieleckig sind. Es ist dabei günstig, die Seitenwände der Eingriffsstruktur 19 und des Eingriffselements 25 parallel zu der Drehachse A auszurichten, um eine gute Übertragung des von dem Antrieb 21 eingebrachten Drehmoments zu gewährleisten. Auch eine inverse Gestaltung, bei der zum Beispiel die Eingriffsstruktur 19 als Zapfen und das Eingriffselement 25 als zum Zapfen passende Nut ausgebildet ist, sind problemlos möglich.

Werden die Sterileinheit 10 und die Instrumentenantriebseinheit 20 miteinander verbunden, sind die Eingriffsstruktur 19 und das Eingriffselement 25 in der Regel nicht zueinander ausgerichtet und greifen nicht ineinander. Die Übertragung eines Drehmoments und somit das Antreiben des chirurgischen Instruments 30 ist auf diese Weise nicht möglich. Das Übertragungselement 12 liegt dabei mit einer Unterseite auf dem Eingriffselement 25 auf, so dass es angehoben wird und sich in der ersten Endlage befindet. In der ersten Endlage stehen die zueinander komplementären konischen Bereiche 18a, 18b form- und kraftschlüssig in Verbindung.

Um ein Drehmoment von einem Motor 24 des Antriebs 21 der Instrumentenantriebseinheit 20 auf das Übertragungselement 12 übertragen zu können, muss nun das Eingriffselement 25 zur Eingriffsstruktur 19 ausgerichtet werden, so dass diese ineinandergreifen. Dies wird durch die konischen Bereiche 18a, 18b bewerkstelligt, die in der ersten Endlage durch den Form- und Kraftschluss selbsthemmend wirken. Mittels unterschiedlicher Reibungskoeffizienten und Reibradien zwischen Eingriffselement 25 und Eingriffsstruktur 19 kann dieser Effekt weiter verstärkt werden. Das System ist allgemein so ausgelegt, dass ein erstes Reibmoment, welches zwischen dem Eingriffselement 25 und der Unterseite des Übertragungselements 12 wirkt, geringer als ein zweites Reibmoment ist, welches zwischen den konischen Bereichen 18a, 18b wirkt. Beschreibt man die konischen Bereiche 18a, 18b mit Hilfe eines Kegelstumpfs, so weist der Kegel, aus welchem der Kegelstumpf entnommen ist, einen Öffnungswinkel auf. Umso spitzer dieser Öffnungswinkel wird, desto größer wird das erste Reibmoment. Wird ein kritischer Öffnungswinkel unterschritten, der erfahrungsgemäß bei ca. 8° - 10° liegt und auch von den gewählten Materialien abhängig ist, kann sich das Übertragungselement 12 nicht ohne äußere Krafteinwirkung aus der ersten Endlage lösen und ein Ausrichten ist nicht mehr möglich. Wird der Öffnungswinkel hingegen größer, reduziert sich das erste Reibmoment, bis der Vorteil durch die konischen Bereiche 18a, 18b erfahrungsgemäß und ebenfalls abhängig von den verwendeten Materialien bei einem Überschreiten von ca. 60° - 80° verloren gehen kann. Zusätzlich kann der Reibungskoeffizient zwischen dem konischen Bereich 18a des Übertragungselements 12 und dem konischen Bereich 18b der Durchgangsöffnung größer als der Reibungskoeffizient zwischen der Unterseite des Übertragungselements 12 und dem Eingriffselement 25 ausgelegt werden. Dies kann damit erreicht werden, dass der konische Bereich 18a des Übertragungselements 12 und/oder der konische Bereich 18b der Durchgangsöffnung aufgeraut oder mit einem den Reibungskoeffizienten vergrößernden Material versehen sind, während die Unterseite des Übertragungselements 12 und das Eingriffselement 25 glatt sind.

Stehen die zueinander komplementären konischen Bereiche 18a, 18b form- und kraftschlüssig in Verbindung, kommt es entweder dazu, dass sich das Übertragungselement 12 nicht um die Drehachse A rotiert, während das Eingriffselement 25 über die Unterseite des Übertragungselements 12 gleitet. Alternativ kommt es dazu, dass sich das Übertragungselement 12 langsamer als das Eingriffselement 25 dreht. In beiden Fällen ist die Drehung des Übertragungselements 12 um die Drehachse A relativ zu der Basis 11 gehemmt und das Eingriffselement 25 wird durch eine Drehung mittels des Motors 24 beständig in seiner Lage zu dem Übertragungselement 12 angepasst, da es schneller als das Übertragungselement 12 dreht.

Wird nun eine Position erreicht, bei der das Übertragungselement 12 und das Eingriffselement 25 rotatorisch um die Drehachse A so zueinander ausgerichtet sind, dass das Eingriffselement 25 in die Eingriffsstruktur 19 greift, fällt das Übertragungselement 12 in die zweite Endlage. Das Eingriffselement 25 wird dabei von der Eingriffsstruktur 19 aufgenommen, so dass ein an dem Eingriffselement 25 senkrecht zur Drehachse A wirkendes Drehmoment auf die Eingriffsstruktur 19 und somit auf das Übertragungselement 12 übertragen wird. Da die konischen Bereiche 18a, 18b nicht mehr form- und kraftschlüssig verbunden sind, wirkt an dieser Stelle nun kein Reibmoment mehr und das Übertragungselement 12 kann frei um die Drehachse A rotieren und das Drehmoment nahezu verlustfrei übertragen.

### Bezugszeichenliste

- 10: Sterileinheit
- 11: Basis
- 12: Übertragungselement
- 13: erster Befestigungsmechanismus
- 14: zweiter Befestigungsmechanismus
- 15: Befestigungsfläche
- 16: Koppelstruktur
- 17: Schnapphaken
- 18a, b: konischer Bereich
- 19: Eingriffsstruktur
- 20: Instrumentenantriebseinheit
- 21: Antrieb
- 22: Antriebsgehäuse
- 23: erstes Befestigungselement
- 24: Motor
- 25: Eingriffselement
- 30: chirurgisches Instrument
- 31: Instrumentengehäuse
- 32: Instrumentenschaft
- 33: Auslösemechanismus
- 35: Endeffektor
- 50: Manipulator
- 60: Sterilbarriere
- A: Drehachse

## Patentansprüche

1. Sterileinheit (10) zum sterilen Verbinden einer Instrumentenantriebseinheit (20) mit einem chirurgischen Instrument (30), umfassend
- eine Basis (11) mit mindestens einer kreisförmigen Durchgangsöffnung, wobei in der Durchgangsöffnung ein Übertragungselement (12) angeordnet ist, welches um eine Drehachse (A) drehbar gelagert und in Richtung der Drehachse (A) axial in einem vorgegebenen Bewegungsbereich zwischen einer ersten und einer zweiten Endlage beweglich ist, und
- einen ersten Befestigungsmechanismus (13), mit dem die Sterileinheit (10) mit der Instrumentenantriebseinheit (20) verbunden wird, und einen zweiten Befestigungsmechanismus (14), mit dem die Sterileinheit (10) mit dem Instrument (30) verbunden wird, **dadurch gekennzeichnet,**
- **dass** das Übertragungselement (12) an einer Umlauffläche und die Durchgangsöffnung an einer Durchgangswand jeweils einen zueinander koaxial ausgerichteten und zueinander komplementären konischen Bereich (18a, 18b) aufweisen,
- die in der ersten Endlage form- und kraftschlüssig in Kontakt stehen, so dass eine Drehung des Übertragungselements (12) um die Drehachse (A) relativ zu der Basis (11) gehemmt wird.

2. Sterileinheit (10) nach Anspruch 1, **dadurch gekennzeichnet, dass**
die konischen Bereiche (18a, 18b) jeweils einen Kegelstumpf eines Kegels bilden, wobei der Kegel einen Öffnungswinkel zwischen 10° und 60° aufweist.

3. Sterileinheit (10) nach Anspruch 1 oder 2, **dadurch gekennzeichnet,**
**dass** der konische Bereich (18a) des Übertragungselements (12) und/oder der konische Bereich (18b) der Durchgangsöffnung aufgeraut oder mit einem den Reibungskoeffizienten vergrößernden Material versehen sind.

4. Sterileinheit (10) nach einem der Ansprüche 1 bis 3, **dadurch gekennzeichnet, dass** das Übertragungselement (12) durch mindestens zwei Schnapphaken (17) in der Durchgangsöffnung gehalten wird.

5. Manipulator für die robotische Chirurgie, umfassend eine Instrumentenantriebseinheit (20) und eine Sterileinheit (10) zum Ankoppeln eines chirurgischen Instruments,
- wobei die Sterileinheit (10) eine Basis (11) mit mindestens einem Übertragungselement (12), das in einer dem mindestens einen Übertragungselement (12) zugeordneten kreisförmigen Durchgangsöffnung angeordnet und welches um eine Drehachse (A) drehbar gelagert und in Richtung der Drehachse (A) axial in einem definierten Bewegungsbereich mit einer ersten und einer zweiten Endlage beweglich ist, und einen ersten Befestigungsmechanismus (13), mit dem die Sterileinheit (10) mit der Instrumentenantriebseinheit (20) verbunden ist, aufweist, und
- wobei die Instrumentenantriebseinheit (20) einen oder mehrere Antriebe (21) aufweist, und dem mindestens einen Übertragungselement (12) ein Antrieb (21) zugeordnet ist,
- wobei der Antrieb (21) ein Eingriffselement (25) und das mindestens eine Übertragungselement (12) eine zu dem Eingriffselement (25) komplementäre Eingriffsstruktur (19) aufweist,
- und sich das Übertragungselement (12) in der zweiten Endlage befindet, wenn das Übertragungselement (12) und das Eingriffselement (25) rotatorisch um die Drehachse (A) so zueinander ausgerichtet sind, dass das Eingriffselement (25) in die Eingriffsstruktur (19) greift und ein an dem Eingriffselement (25) senkrecht zur Drehachse (A) wirkendes Drehmoment auf die Eingriffsstruktur (19) überträgt, und
- das Übertragungselement (12) sich in der ersten Endlage befindet, wenn das Eingriffselement (25) und die Eingriffsstruktur (19) nicht zueinander ausgerichtet sind,
**dadurch gekennzeichnet,**
- **dass** das Übertragungselement (12) an einer Umlauffläche und die Durchgangsöffnung an einer Durchgangswand jeweils einen zueinander koaxial ausgerichteten und zueinander komplementären konischen Bereich (18a, 18b) aufweisen,
- wobei die konischen Bereiche (18a, 18b) in der ersten Endlage formschlüssig und kraftschlüssig in Kontakt kommen, wodurch eine Drehung des Übertragungselements (12) um die Drehachse (A) relativ zu der Basis (11) gehemmt wird.

6. Manipulator nach Anspruch 5, **dadurch gekennzeichnet, dass**
die konischen Bereiche (18a, 18b) jeweils einen Kegelstumpf eines Kegels bilden, wobei der Kegel einen Öffnungswinkel zwischen 10° und 60° aufweist.

7. Manipulator nach Anspruch 5 oder 6, **dadurch gekennzeichnet, dass** der Reibungskoeffizient zwischen dem konischen Bereich (18a) des Übertragungselements (12) und dem konischen Bereich (18b) der Durchgangsöffnung größer als der Reibungskoeffizient zwischen der Unterseite des Übertragungselements (12) und dem Eingriffselement (25) ist.

8. Manipulator nach Anspruch 7, **dadurch gekennzeichnet,**
**dass** der konische Bereich (18a) des Übertragungselements (12) und/oder der konische Bereich (18b) der Durchgangsöffnung aufgeraut oder mit einem den Reibungskoeffizienten vergrößernden Material versehen sind.

9. Manipulator nach einem der Ansprüche 5 bis 8, **dadurch gekennzeichnet, dass** das Übertragungselement (12) durch mindestens zwei Schnapphaken (17) in der Durchgangsöffnung gehalten wird.
